# EUROPEAN PATENT SPECIFICATION

(11) **EP 2 481 344 B1**
(45) Date of publication and mention of the grant of the patent: **19.03.2014**
(21) Application number: 11803425.5
(22) Date of filing: 13.06.2011
(51) Int. Cl.: A61B 1/12, A61B 1/00, G02B 23/24

(54) **ENDOSCOPE CLEANING AND DISINFECTING SYSTEM WITH CONNECTION TOOL AND FLUID SUPPLY APPARATUS**
ENDOSKOP REINIGUNGS- UND DESINFEKTIONSSYSTEM MIT VERBINDUNGSEINRICHTUNG UND FLÜSSIGKEITZUFUHREINRICHTUNG
SYSTEME DE NETTOYAGE ET DE DESINFECTION D'ENDOSCOPE AVEC COUPLEUR ET DISPOSITIF D'ALIMENTATION DE FLUIDE

(30) Priority: 05.07.2010 JP 2010153506
(43) Date of publication of application: 01.08.2012
(73) Proprietor: OLYMPUS MEDICAL SYSTEMS CORP., Tokyo 151-0072 (JP)
(72) Inventor: NAKANISHI Nobuyuki, Tokyo 151-0072 (JP)
(74) Representative: Schicker, Silvia
(86) International application number: PCT/JP2011/063512
(87) International publication number: WO 2012/005089

(56) References cited:
- EP-A2- 0 082 950
- JP-A- 10 234 666
- JP-A- 2004 135 946
- JP-A- 2005 204 827
- JP-A- 2006 334 405
- JP-A- 2009 195 400
- JP-A- 2010 011 977

## Description

### Technical Field

The present invention relates to an endoscope cleaning/disinfecting apparatus connection tool for connecting an endoscope and a fluid supply apparatus, and a fluid supply apparatus.

### Background Art

When a worker cleans and disinfects an inside of endoscope conduits using a cleaning/disinfecting apparatus, the worker first connects a fluid supply apparatus connection portion provided at an end of an endoscope cleaning/disinfecting apparatus connection tool to a fluid supply port of a fluid supply apparatus.

Then, the worker connects an endoscope connection portion provided at another end of the endoscope cleaning/disinfecting apparatus connection tool to a pipe sleeve formed on an outer surface of an endoscope. The pipe sleeve is in communication with the endoscope conduits, such as an air/water delivery conduit and a suction conduit, provided inside the endoscope.

As a result, fluids, such as a cleaning solution, a disinfectant solution, rinse water and/or a gas, are supplied from the fluid supply apparatus to the inside of the endoscope conduits via the endoscope cleaning/disinfecting apparatus connection tool and an opening portion of the pipe sleeve, whereby the inside of the endoscope conduits is cleaned/disinfected.

Japanese Patent Application Laid-Open Publication No. 2004-135946 discloses an endoscope cleaning/disinfecting apparatus connection tool including an endoscope connection portion that includes a first flow channel including a first opening for supplying a fluid to an endoscope conduit via a pipe sleeve, and a second flow channel including a second opening for supplying a fluid to an outer surface of the pipe sleeve.

The connection tool disclosed in Japanese Patent Application Laid-Open Publication No. 2004-135946 has a configuration for supplying the fluid only from the first opening when performing detection of clogging in the endoscope conduit or when supplying the fluid only to the endoscope conduit with the second flow channel closed by inflating a balloon provided in the second flow channel, and supplying the fluid from the second opening as well as the first opening when it is desired to clean/disinfect the pipe sleeve as well as the endoscope conduit by shrinking the balloon, and with this configuration the endoscope conduit can be cleaned/disinfected with the same amount of fluid as the conventional art and also the detection of clogging can be performed.

However, using the endoscope cleaning/disinfecting apparatus connection tool disclosed in Japanese Patent Application Laid-Open Publication No. 2004-135946, when performing cleaning/disinfecting of the pipe sleeve as well as the endoscope conduits in the state where the balloon is shrunk, the fluid is merely leaked from the second opening according to the configuration in Patent Document 1, and therefore there has been a problem that the cleaning can not be positively performed although the disinfection can be sufficiently performed.

Further, since in Japanese Patent Application Laid-Open Publication No. 2004-135946 there is not provided a configuration for closing the first opening, there has been a problem that the fluid can not be discharged only from the second opening, and thus it is impossible to positively perform cleaning of the pipe sleeve.

The present invention has been made in view of the above problems and an object of the present invention is to provide an endoscope cleaning/disinfecting apparatus connection tool and a fluid supply apparatus having configurations capable of cleaning/disinfecting an inside of an endoscope conduit and also positively cleaning/disinfecting a pipe sleeve of the endoscope.

EP 0 082 950 A2, on which document is based the preamble of claim 1, discloses a liquid supply device for an endoscope comprising a connecting member detachably connectable to a connector of the endoscope, a liquid supply tube connected at one end to the connecting member and located at the other end in a liquid supply tank, and an air supply tube connected at one end to the connecting member and at the other end to an air supply pump, causing pressure in the liquid supply tank to be increased by air from the air supply pump to permit the liquid within the tank to be sent to the liquid supply tube.

### Disclosure of Invention

### Means for Solving the Problem

The present invention is defined in claim 1.

### Brief Description of the Drawings

Fig. 1 is a diagram illustrating an endoscope cleaning/disinfecting apparatus connection tool according to the present embodiment, together with an endoscope and a fluid supply apparatus;
Fig. 2 is a diagram illustrating a state in which the endoscope cleaning/disinfecting apparatus connection tool in Fig. 1 is connected to a pipe sleeve of an endoscope and a port of a fluid supply apparatus;
Fig. 3 is a table indicating on/off control of two solenoid valves performed by a control section in Fig. 1 for respective steps in a cleaning/disinfecting process;
Fig. 4 is a diagram illustrating an example useful for understanding the present invention in which a second fluid passage portion of the endoscope cleaning/disinfecting apparatus connection tool in Fig. 1 is provided outside a first fluid passage portion abreast of the first fluid passage portion;
Fig. 5 is a diagram illustrating a state in which an endoscope connection portion of an endoscope cleaning/disinfecting apparatus connection tool is connectable to a treatment instrument insertion pipe sleeve provided at an operation section of an endoscope;
Fig. 6 is a diagram partially illustrating a state in which an endoscope connection portion of an endoscope cleaning/disinfecting apparatus connection tool is threadedly connected to a pipe sleeve of an endoscope;
Fig. 7 is a diagram illustrating a state in which an endoscope cleaning/disinfecting apparatus connection tool is connected to an LG connector of an endoscope and a port of a fluid supply apparatus;
Fig. 8 is a diagram illustrating a state in which a cap is connected to a pipe sleeve of an endoscope;
Fig. 9 is a diagram illustrating a state in which an endoscope cleaning/disinfecting apparatus connection tool is connected to each of an LG connector, and a treatment instrument insertion pipe sleeve, of an endoscope, and a cap is connected to the pipe sleeve of the endoscope; and
Fig. 10 is a perspective view illustrating an example of an endoscope cleaning/disinfecting apparatus together with an endoscope.

### Best Mode for Carrying Out the Invention

Hereinafter, an embodiment of the present invention will be described with reference to the drawings. It should be noted that the drawings are schematic ones and the relationship between the thickness and the width of each member, the ratios in thickness between the respective members, and the like, may be different from actual ones, and it should be understood that there may be differences between the respective drawings in terms of dimensions and ratios.

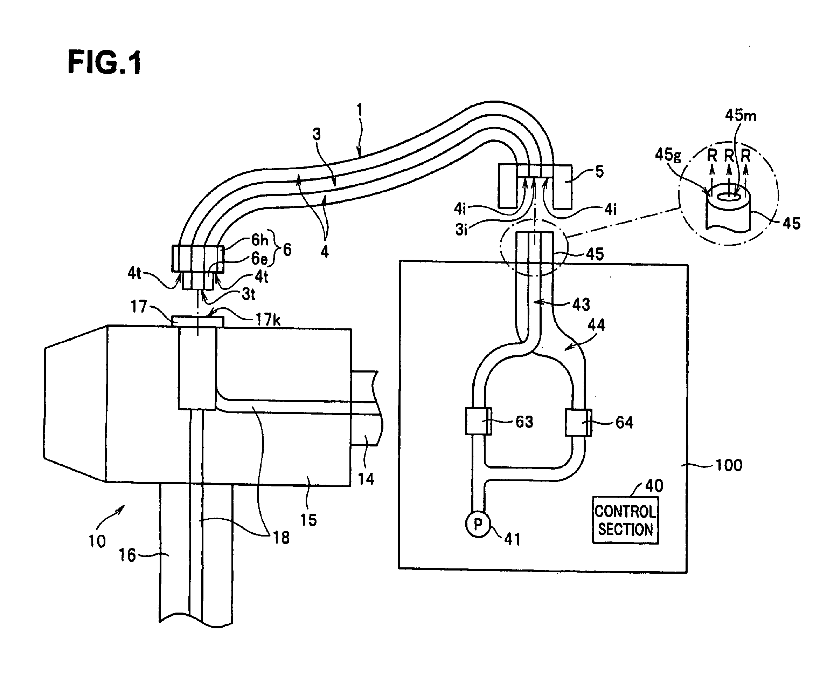

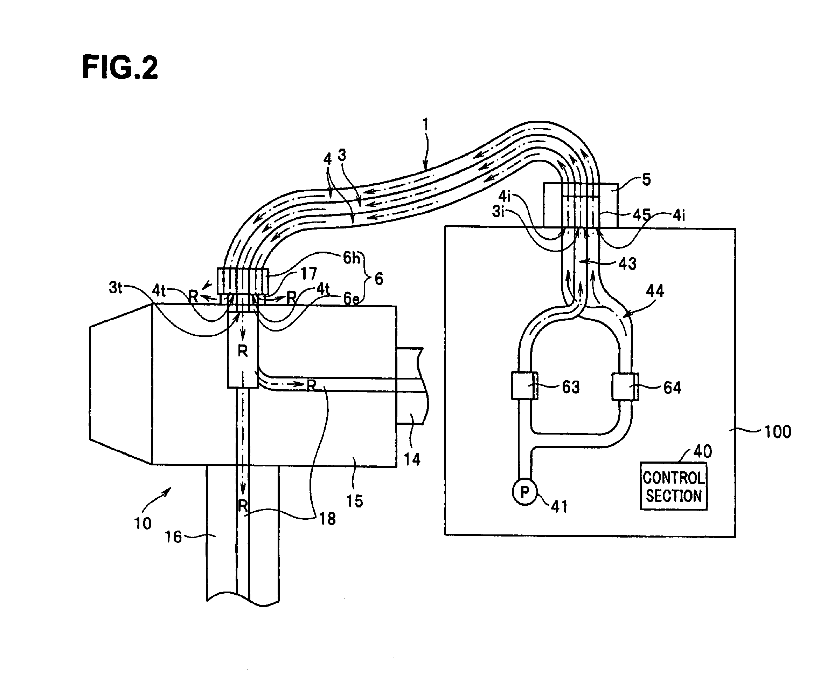

Fig. 1 is a diagram illustrating an endoscope cleaning/disinfecting apparatus connection tool according to the present embodiment together with an endoscope and a fluid supply apparatus, and Fig. 2 is a diagram illustrating a state in which the endoscope cleaning/disinfecting apparatus connection tool in Fig. 1 is connected to a pipe sleeve of an endoscope and a port of a fluid supply apparatus.

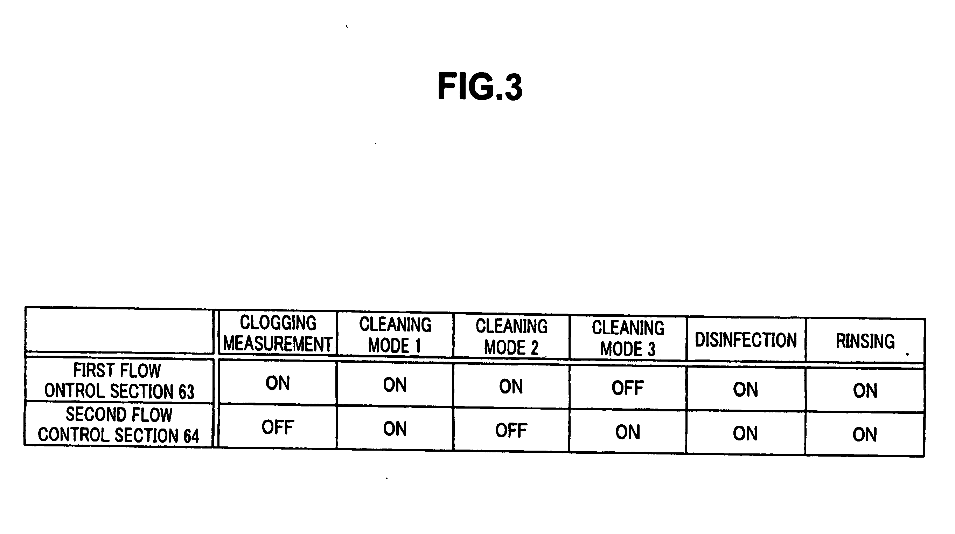

Also, Fig. 3 is a table indicating on/off control of two solenoid valves performed by a control section in Fig. 1 for respective steps in a cleaning and disinfecting process.

As illustrated in Fig. 1, an endoscope cleaning/disinfecting apparatus connection tool 1 is provided to connect an endoscope 10 and a fluid supply apparatus 100, and includes a main part that includes a fluid supply apparatus connection portion 5, a first fluid passage portion 3, a second fluid passage portion 4, and an endoscope connection portion 6.

The fluid supply apparatus connection portion 5 is provided at respective ends of the first fluid passage portion 3 and the second fluid passage portion 4, and an introduction port 3i and an introduction port 4i provided at respective ends of the first fluid passage portion 3 and the second fluid passage portion 4 are opened to the fluid supply apparatus connection portion 5.

Furthermore, the fluid supply apparatus connection portion 5 is connectable to a port 45 of the fluid supply apparatus 100 as illustrated in Figs. 1 and 2. After the fluid supply apparatus connection portion 5 is connected to the port 45, the introduction ports 3i and 4i come into communication with a first conduit 43 and a second conduit 44, which will be described later, of the fluid supply apparatus via supply ports 45m and 45g, respectively.

The endoscope connection portion 6 is provided at respective other ends of the first fluid passage portion 3 and the second fluid passage portion 4, and is connectable to a pipe sleeve 17 of an operation section 15, the pipe sleeve 17 including an opening portion 17k that is in communication with endoscope conduits 18 extending in an insertion portion 14, the operation section 15 and a universal cord 16 in the endoscope 10 as illustrated in Figs. 1 and 2. Examples of the endoscope conduits 18 may include a known suction conduit and a known air/water delivery conduit.

Furthermore, as illustrated in Fig. 2, the endoscope connection portion 6 includes a projection portion 6e that is inserted into the pipe sleeve 17 via the opening portion 17k when the endoscope connection portion 6 is connected to the pipe sleeve 17, and the projection portion 6e includes a first discharge portion 3t, which is an opening on the other end side of the first fluid passage portion 3. As a result of the projection portion 6e being inserted into the pipe sleeve 17, the endoscope connection portion 6 is positioned relative to the pipe sleeve 17.

As illustrated in Fig. 2, the first discharge portion 3t discharges a fluid R supplied from the fluid supply apparatus 100 and passed through the first fluid passage portion 3, to the inside of the endoscope conduit 18 when the endoscope connection portion 6 is connected to the pipe sleeve 17.

Furthermore, as illustrated in Fig. 2, the endoscope connection portion 6 includes a body portion 6h that is located outside the pipe sleeve 17 when the endoscope connection portion 6 is connected to the pipe sleeve 17, the body portion 6h including a second discharge portion 4t, which is an opening on the other end side of the second fluid passage portion 4.

As illustrated in Fig. 2, the second discharge portion 4t is arranged at a circumference of the opening portion 17k of the pipe sleeve 17 when the endoscope connection portion 6 is connected to the pipe sleeve 17, and discharges the fluid R supplied from the fluid supply apparatus 100 and passed through the second fluid passage portion 4 to the circumference of the opening portion 17k, that is, an outer surface of the pipe sleeve 17.

The first fluid passage portion 3 and the second fluid passage portion 4 are provided to allow the fluid R supplied from the fluid supply apparatus 100 via the port 45 to pass through, and each are formed of, for example, a tubular member.

Furthermore, the second fluid passage portion 4 has a diameter larger than a diameter of the first fluid passage portion 3, and the first fluid passage portion 3 is arranged inside the second fluid passage portion 4 along the second fluid passage portion 4. In other words, the first fluid passage portion 3 and the second fluid passage portion 4 jointly form a known coaxial tube.

The fluid supply apparatus 100 includes the first conduit 43 that comes into communication with the first fluid passage portion 3 when the fluid supply apparatus connection portion 5 is connected to the port 45, the first conduit 43 supplying the fluid R to the first fluid passage portion 3 via the introduction port 3i, and the second conduit 44 that comes into communication with the second fluid passage portion 4 when the fluid supply apparatus connection portion 5 is connected to the port 45, the second conduit 44 supplying the fluid R to the second fluid passage portion 4 via the introduction port 4i.

Furthermore, the second conduit 44 has a diameter larger than a diameter of the first conduit 43 in a region downstream of a first flow control section 63 and a second flow control section 64, which will be described later, and the first conduit 43 is located inside the second conduit 44 in the region downstream of the first flow control section 63 and the second flow control section 64.

As illustrated in, for example, Fig. 1, the port 45 includes the supply ports 45m and 45g so that the fluid R supplied from the first conduit 43 and the fluid R from the second conduit 44 can be supplied through one port to the first fluid passage portion 3 and the second fluid passage portion 4 via the introduction ports 3i and 4i, respectively.

The first flow control section 63 is provided at a position part way through the first conduit 43. Also, the second flow control section 64 is provided at a position part way through the second conduit 44. The first flow control section 63 and the second flow control section 64 each include, for example, an openabte/closable solenoid valve. Furthermore, operation to open/close the first flow control section 63 and the second flow control section 64 is controlled by a control section 40 provided in the fluid supply apparatus 100.

Furthermore, the first conduit 43 is in communication with the second conduit 44 in a region upstream of the first flow control section 63 and the second flow control section 64, and a pump 41 for fluid supply is provided in the region upstream of the communication portion. Driving of the pump 41 is controlled by the control section 40. Also, the pump 41 may be a self-suction pump that can deliver a gas.

Although the configuration upstream of the pump 41 is omitted for simplicity of illustration in Figs. 1 and 2, a conduit provided upstream of the pump 41 is in communication with tanks each storing, e.g., a cleaning solution, a disinfectant solution and rinse water.

As a result, when the pump 41 is driven, a fluid R such as a cleaning solution, a disinfectant solution, rinse water or a gas is supplied selectively or simultaneously to the first fluid passage portion 3 and the second fluid passage portion 4 via the first conduit 43 and the second conduit 44 according to whether the first flow control section 63 and the second flow control section 64 are in an on (opened) or an off (closed) state.

The control section 40 performs control to open/close the first flow control section 63 and the second flow control section 64, and thereby performs control to supply the fluid R to at least one of the first conduit 43 and the second conduit 44. Furthermore, the control section 40 has a function for controlling an amount of supply of the fluid R flowing in the first conduit 43 and an amount of supply of the fluid R flowing in the second conduit 44 by means of controlling the time during which the first flow control section 63 and the second flow control section 64 are opened and the time during which the pump 41 is driven.

Hereinafter, a method for controlling the first flow control section 63 and the second flow control section 64 performed by the control section 40 will be described in details with reference to Fig. 3. Here, it is assumed that when the control section 40 controls the first flow control section 63 and the second flow control section 64, as illustrated in Fig. 2, the endoscope connection portion 6 of the endoscope cleaning/disinfecting apparatus connection tool 1 is connected to the pipe sleeve 17 and the fluid supply apparatus connection portion 5 of the endoscope cleaning/disinfecting apparatus connection tool 1 is connected to the port 45.

As illustrated in Fig. 3, first, in a case of detecting clogging inside the endoscope conduit 18, the control section 40 performs control to turn on the first flow control section 63 and also performs control to turn off the second flow control section 64.

Consequently, a fluid supplied as a result of driving of the pump 41 passes through the first conduit 43, the supply port 45m, the introduction port 3i and the first fluid passage portion 3 and is discharged from the first discharge portion 3t only to the inside of the endoscope conduit 18 because only the first flow control section 63 is in an on state.

Here, since the second flow control section 64 is in an off state, no fluid is discharged from the second discharge portion 4t, and thus, the fluid is intensively supplied only to the inside of the endoscope conduit 18, enabling correct detection of clogging inside the endoscope conduit 18.

Next, when normal cleaning of the endoscope 10 is performed (cleaning mode 1), when the endoscope 10 is disinfected after cleaning, and furthermore, when the endoscope 10 is rinsed, the control section 40 performs control to turn on both the first flow control section 63 and the second flow control section 64.

As a result, any of the cleaning solution, the disinfectant solution and the rinse water supplied as a result of driving of the pump 41 passes through the first conduit 43, the supply port 45m, the introduction port 3i and the first fluid passage portion 3 and is discharged from the first discharge portion 3t to the inside of the endoscope conduit 18 because the first flow control section 63 is in an on state, and also passes through the second conduit 44, the supply port 45g, the introduction port 4i and the first fluid passage portion 4 and is discharged from the second discharge portion 4t to the circumference of the opening portion 17k of the pipe sleeve 17 because the second flow control section 64 is also in an on state.

Consequently, while the inside of the endoscope conduit 18 is cleaned, disinfected or rinsed, the pipe sleeve 17 is actively cleaned, disinfected or rinsed by means of the cleaning solution, the disinfectant solution or the rinse water discharged from the second discharge portion 4t. In this case, not only the pipe sleeve 17, but also an outer surface of the projection portion 6e of the endoscope connection portion 6 is cleaned, disinfected or rinsed.

Meanwhile, when only the inside of the endoscope conduit 18 is actively cleaned (cleaning mode 2), for example, when only the inside of the endoscope conduit 18 is actively cleaned and disinfected by means of high-pressure cleaning or supply of a known two-phase gas-liquid flow, the control section 40 performs control to turn on the first flow control section 63 and turn off the second flow control section 64 as in the case of clogging detection.

As a result, for example, the two-phase gas-liquid flow or the high-pressure fluid supplied as a result of driving of the pump 41 passes through the first conduit 43, the supply port 45m, the introduction port 3i and the first fluid passage portion 3 and is intensively discharged from the first discharge portion 3t only to the inside of the endoscope conduit 18 because only the first flow control section 63 is in an on state. Thus, only the endoscope conduit 18 is actively cleaned. An operation similar to the above operation is performed for a case where only the inside of the endoscope conduit 18 is disinfected or rinsed.

Furthermore, when only the pipe sleeve 17 is intensively cleaned (cleaning mode 3), the control section 40 performs control to turn on only the second flow control section 64 and turn off the first flow control section 63.

Consequently, the cleaning solution as a result of driving of the pump 41 passes though the second conduit 44, the supply port 45g, the introduction port 4i and the first fluid passage portion 4 and is discharged from the second discharge portion 4t to the circumference of the opening portion 17k because only the second flow control section 64 is in an on state.

Accordingly, since the first flow control section 63 is in an off state, no cleaning solution is discharged from the first discharge portion 3t, and thus, the pipe sleeve 17 is actively and intensively cleaned by the cleaning solution discharged from the second discharge portion 4t. An operation similar to the above operation is performed for a case where only the pipe sleeve 17 is disinfected or rinsed.

It has been described above that in the present embodiment, the endoscope cleaning/disinfecting apparatus connection tool 1 is configured so that, when the endoscope connection portion 6 is connected to the pipe sleeve 17, the first discharge portion 3t of the first fluid passage portion 3 is arranged inside the pipe sleeve 17 and discharges the fluid R to the endoscope conduit 18, and the second discharge portion 4t of the second fluid passage portion 4 is arranged at the circumference of the opening portion 17k of the pipe sleeve 17 and discharges the fluid R to the circumference of the opening portion 17k.

While in a conventional configuration, a fluid is supplied to a circumference of an opening portion 17k by means of the fluid being run out from the pipe sleeve 17, and thus, in order to clean the circumference of the opening portion 17k, it is necessary to increase the amount of fluid to be supplied, requiring a long time for the cleaning, in the configuration according to the present embodiment, the fluid R supplied via the second conduit 44 and the second fluid passage portion 4 as a result of the second flow control section 64 being turned on is exclusively and actively spayed to the circumference of the opening portion 17k from the second discharge portion 4t, enabling the circumference of the opening portion 17k to be cleaned in a short period of time.

Furthermore, since only the first discharge portion 3t is arranged inside the pipe sleeve 17, the fluid R supplied via the first conduit 43 and the first fluid passage portion 3 as a result of the first flow control section 63 being turned on is exclusively supplied to the inside of the endoscope conduit 18 from the first discharge portion 3t, enabling the inside of the endoscope conduit 18 to be cleaned in a short period of time.

Furthermore, it has been described that in the present embodiment, the control section 40 performs control to open/close the first flow control section 63 and the second flow control section 64, and also performs control the time periods during which the first flow control section 63 and the second flow control section 64 are opened.

Consequently, time periods during which the endoscope conduit 18 and the pipe sleeve 17 are cleaned/disinfected can be separately controlled, as well as the amount of supply of the fluid R to the endoscope conduit 18 and the amount of supply of the fluid R to the pipe sleeve 17 can be separately adjusted. Furthermore, the fluid R can be supplied selectively or simultaneously to the endoscope conduit 18 and the pipe sleeve 17, and thus, the endoscope conduit 18 and/or the pipe sleeve 17 can be actively cleaned/disinfected depending on the purpose of use. Thus, for example, cleaning/disinfection of only the pipe sleeve 17, which could not be performed conventionally, can easily be performed.

According to the above, the endoscope cleaning/disinfecting apparatus connection tool 1 and the fluid supply apparatus 100 that are configured to clean/disinfect the inside of the endoscope conduit 18 and actively clean/disinfect the pipe sleeve 17 of the endoscope 10 can be provided.

The fluid supply apparatus 100 proposed herein may be one that adjusts cleaning/disinfecting time of the pipe sleeve 17 and cleaning/disinfecting time of the endoscope conduit 18 according to the type and/or state of the endoscope. For example, an adjustment can be made to increase cleaning time or disinfecting time of the pipe sleeve 17 for endoscopes whose pipe sleeve portions generally easily get dirty. An adjustment can also be made to decrease time for cleaning or disinfecting a portion that is hard to get dirty.

Also, the fluid supply apparatus 100 proposed herein may have a function for reading information provided to an endoscope such as an RFID reading function, for example.

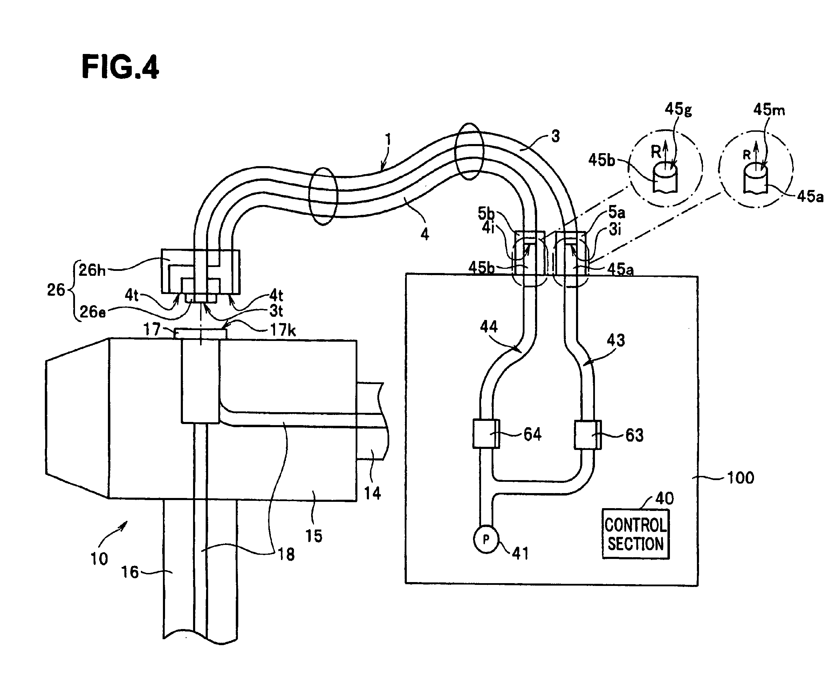

An example useful for understanding the present invention will be described below with reference to Fig. 4. Fig. 4 is a diagram illustrating a modified example in which the second fluid passage portion of the endoscope cleaning/disinfecting apparatus connection tool in Fig. 1 is provided outside the first fluid passage portion abreast of the first fluid passage portion.

It has been described above that in the endoscope cleaning/disinfecting apparatus connection tool 1 of the present embodiment, the second fluid passage portion 4 has a diameter larger than the diameter of the first fluid passage portion 3, and the first fluid passage portion 3 is provided inside the second fluid passage portion 4.

As illustrated in Fig. 4, a first fluid passage portion 3 may be arranged outside a second fluid passage portion 4 abreast of the second fluid passage portion 4.

More specifically, in an endoscope cleaning/disinfecting apparatus connection tool 1, a fluid supply apparatus connection portion 5a is provided at an end of the first fluid passage portion 3, and an introduction port 3i provided at an end of the first fluid passage portion 3 is opened to the fluid supply apparatus connection portion 5a.

Also, a fluid supply apparatus connection portion 5b is provided at an end of the second fluid passage portion 4, and an introduction port 4i provided at an end of the second fluid passage portion 4 is opened to the fluid supply apparatus connection portion 5b.

The fluid supply apparatus connection portions 5a and 5b are connectable to ports 45a and 45b of a fluid supply apparatus 100. After the fluid supply apparatus connection portions 5a and 5b are connected to the ports 45a and 45b, the introduction ports 3i and 4i come into communication with a first conduit 43 and a second conduit 44 of the fluid supply apparatus via supply ports 45m and 45g, respectively. The supply port 45m is in communication with the first conduit 43, and the supply port 45g is in communication with the second conduit 44.

In the endoscope cleaning/disinfecting apparatus connection tool 1, an endoscope connection portion 26 is provided at respective other ends of the first fluid passage portion 3 and the second fluid passage portion 4, and the endoscope connection portion 26 is connectable to a pipe sleeve 17.

Furthermore, the endoscope connection portion 26 includes a projection portion 26e that is inserted into the pipe sleeve 17 via an opening portion 17k when the endoscope connection portion 26 is connected to the pipe sleeve 17, and the projection portion 26e includes a first discharge portion 3t, which is an opening on the other end side of the first fluid passage portion 3. As a result of the projection portion 26e being inserted into the pipe sleeve 17, the endoscope connection portion 26 is positioned relative to the pipe sleeve 17.

Furthermore, the endoscope connection portion 26 includes a body portion 26h that is located outside the pipe sleeve 17 when the endoscope connection portion 26 is connected to the pipe sleeve 17, the body portion 26h including a second discharge portion 4t, which is an opening on the other end side of the second fluid passage portion 4.

The first fluid passage portion 3 and the second fluid passage portion 4 are provided to allow a fluid R supplied from the fluid supply apparatus 100 via the ports 45a and 45b, respectively, to pass through, and each are formed of, for example, a tubular member. Furthermore, the second fluid passage portion 4 is provided outside the first fluid passage portion 3 abreast of the first fluid passage portion 3.

As described above, effects similar to those of the present embodiment described above can be provided even when the first fluid passage portion 3 and the second fluid passage portion 4 are provided separately from each other.

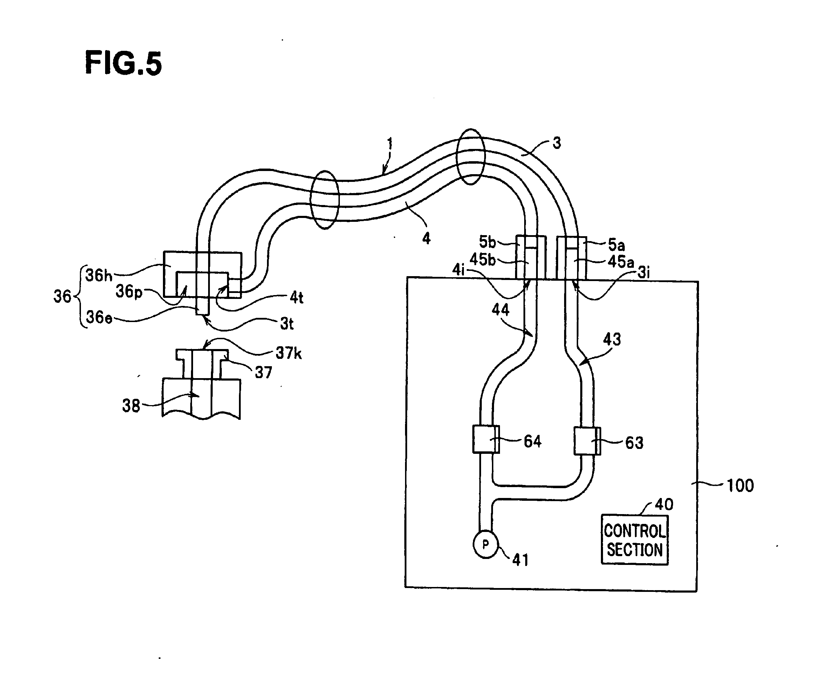

A modified example of the configuration in Fig. 4 will be described with reference to Fig. 5. Fig. 5 is a diagram illustrating a state in which an endoscope connection portion of an endoscope cleaning/disinfecting apparatus connection tool is connectable to a treatment instrument insertion pipe sleeve provided at an operation section of an endoscope.

It has been described that in the present embodiment described above with reference to Figs. 1 to 3 and the example useful for understanding the present invention illustrated in Fig. 4, the endoscope connection portion 6 or 26 of the endoscope cleaning/disinfecting apparatus connection tool 1 is detachably connected to the pipe sleeve 17 provided in the operation section 15 of the endoscope 10.

The configuration of the present invention is not limited to the configurations of the embodiment and the example above, and as illustrated in Fig. 5, an endoscope connection portion 36 may be connectable to a pipe sleeve 37 of a treatment instrument insertion conduit 38 provided inside the endoscope 10, which is provided on the insertion portion 14 side relative to the pipe sleeve 17 in the operation section.

More specifically, as illustrated in Fig. 5, an endoscope connection portion 36 is provided at respective other ends of a first fluid passage portion 3 and a second fluid passage portion 4 of an endoscope cleaning/disinfecting apparatus connection tool 1, and the endoscope connection portion 36 is connectable to the pipe sleeve 37 of the treatment instrument insertion conduit 38.

Furthermore, the endoscope connection portion 36 includes a projection portion 36e that is inserted into the pipe sleeve 37 via an opening portion 37k when the endoscope connection portion 36 is connected to the pipe sleeve 37, and the projection portion 36e includes a first discharge portion 3t, which is an opening on the other end side of the first fluid passage portion 3. As a result of the projection portion 36e being inserted into the pipe sleeve 37, the endoscope connection portion 36 is positioned relative to the pipe sleeve 37.

Furthermore, the endoscope connection portion 36 includes a recess portion 36p formed on the projection portion 36e side of a body portion 36h that is located outside the pipe sleeve 37 when the endoscope connection portion 36 is connected to the pipe sleeve 37, and the recess portion 36p includes a second discharge portion 4t, which is an opening on the other end side of the second fluid passage portion 4.

The recess portion 36p covers an outer circumference of the pipe sleeve 37 when the endoscope connection portion 36 is connected to the pipe sleeve 37, whereby a fluid R discharged from the second discharge portion 4t is supplied to an outer surface of the pipe sleeve 37.

The rest of configuration is the same as that in Fig. 4. The present configuration is applicable to the configuration illustrated in Fig. 1 in which the first fluid passage portion 3 is arranged inside the second fluid passage portion 4.

As described above, the endoscope cleaning/disinfecting apparatus connection tool 1 is also connectable to the pipe sleeve 37 of the treatment instrument insertion conduit 38.

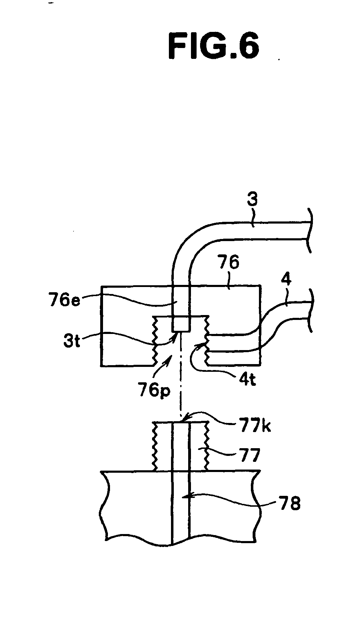

Another example useful for understanding the present invention will be described with reference to Fig. 6. Fig. 6 is a diagram partially illustrating a state in which an endoscope connection portion of an endoscope cleaning/disinfecting apparatus connection tool is threadedly connected to a pipe sleeve of an endoscope.

As illustrated in Fig. 6, when a male thread is provided at an outer circumferential surface of a pipe sleeve 77 including an opening portion 77k of an endoscope conduit 78 provided in an operation section 15 of an endoscope 10, the endoscope cleaning/disinfecting apparatus connection tool 1 only needs to include the configuration described below.

In the endoscope cleaning/disinfecting apparatus connection tool 1, an endoscope connection portion 76 is provided at respective other ends of a first fluid passage portion 3 and a second fluid passage portion 4, and the endoscope connection portion 76 is connectable to the pipe sleeve 77.

More specifically, the endoscope connection portion 76 includes a recess portion 76p on the pipe sleeve 77 side, the recess portion 76p covering the pipe sleeve 77 when the endoscope connection portion 76 is connected to the pipe sleeve 77, and a female thread is provided at an inner circumferential surface of the recess portion 76p, the female thread being engaged with the male thread provided at the outer circumferential surface of the pipe sleeve 77 when the endoscope connection portion 76 is connected to the pipe sleeve 77.

Furthermore, the recess portion 76p includes a second discharge portion 4t, which is an opening on the other end side of the second fluid passage portion 4.

When the endoscope connection portion 76 is connected to the pipe sleeve 77, the recess portion 76p covers the outer circumference of the pipe sleeve 77, whereby a fluid R discharged from the second discharge portion 4t is supplied to an outer surface of the pipe sleeve 77. Accordingly, the part of engagement between the male thread and the female thread, which is conventionally hard to clean when a threaded connection is employed, can be cleaned/disinfected by the fluid R discharged from the second discharge portion 4t.

Furthermore, the recess portion 76p includes a projection portion 76e that is inserted into the pipe sleeve 77 via the opening portion 77k when the endoscope connection portion 76 is connected to the pipe sleeve 77, and the projection portion 76e includes a first discharge portion 3t, which is an opening on the other end side of the first fluid passage portion 3. As a result of the projection portion 76e being inserted into the pipe sleeve 77, the endoscope connection portion 76 is positioned relative to the pipe sleeve 77.

As described above, the endoscope cleaning/disinfecting apparatus connection tool 1 is also connectable to the pipe sleeve 77 to which the endoscope connection portion 76 is threadedly connected.

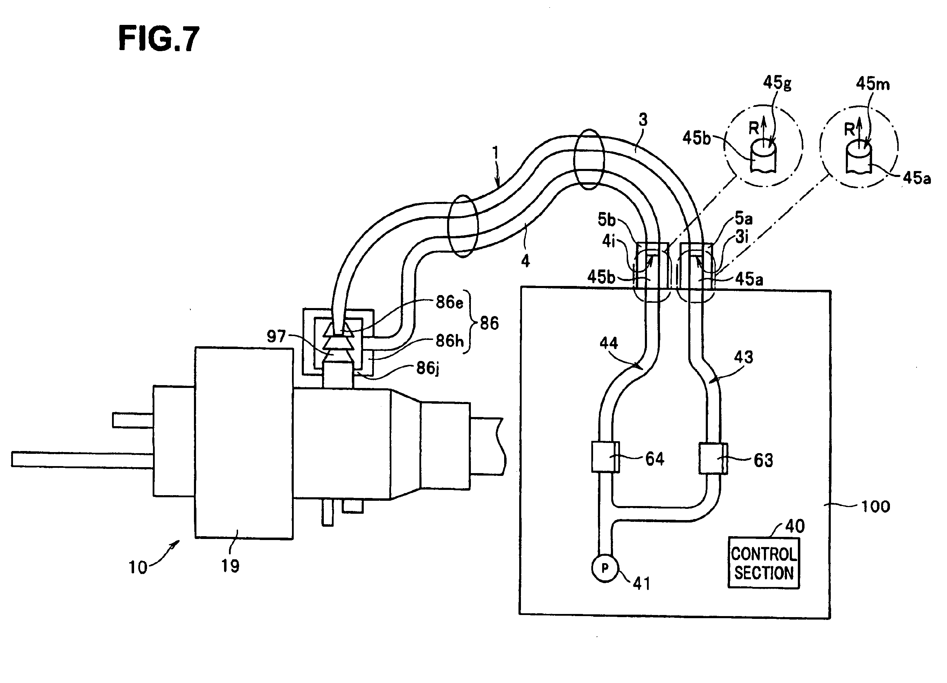

Furthermore, as illustrated in Fig. 7, an endoscope connection portion 86 may be connectable to a pipe sleeve 97 provided at a light guide connector (LG connector) 19.

Fig. 7 illustrates an example useful for understanding the present invention using the endoscope connection portion 86 including a body portion 86h provided with a hook portion 86j for making the body portion 86h be hard to come off from the pipe sleeve. However, the endoscope connection portion 6 illustrated in Fig. 1, the endoscope connection portion 26 illustrated in Fig. 4 or the endoscope connection portion 36 illustrated in Fig. 5 may be connected to the pipe sleeve 97 of the LG connector 19.

Alternatively, the endoscope connection portion 86 illustrated in Fig. 7 may be connected to the pipe sleeve 17 or the pipe sleeve 37.

The hook portion 86j of the endoscope connection portion 86 may have a structure that closely adhere to an entire circumference of the pipe sleeve 97, a claw-like structure or a sponge-like structure. Where the hook portion 86j has the structure that closely adheres to the entire circumference of the pipe sleeve 97, it is desirable that a hole through which a liquid can escape be provided somewhere in the body portion 86h or the hook portion 86j.

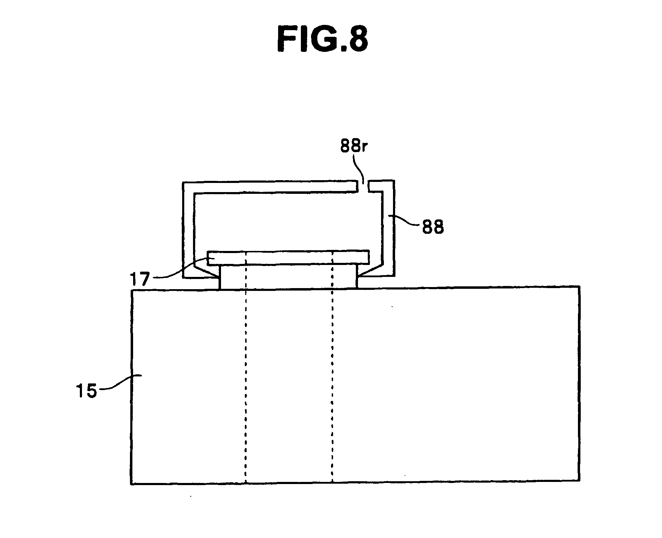

Furthermore, where a fluid is supplied from the pipe sleeve 97 of the LG connector 19, it is preferable that the pipe sleeve 17 of an operation section 15 be occluded or covered by a cap 88 that reduces an amount of discharge as illustrated in Fig. 8. The cap 88 reduces the amount of discharge of a liquid by making a hole 88r from which the liquid escapes has a diameter smaller than a diameter of an opening of the pipe sleeve 17, however, the shape of the cap is not limited to the shape of this cap.

Where a fluid is supplied from the pipe sleeve 97, the amount of liquid flowing on an endoscope insertion portion 14 side can be maintained by reducing the amount of discharge from the pipe sleeve 17.

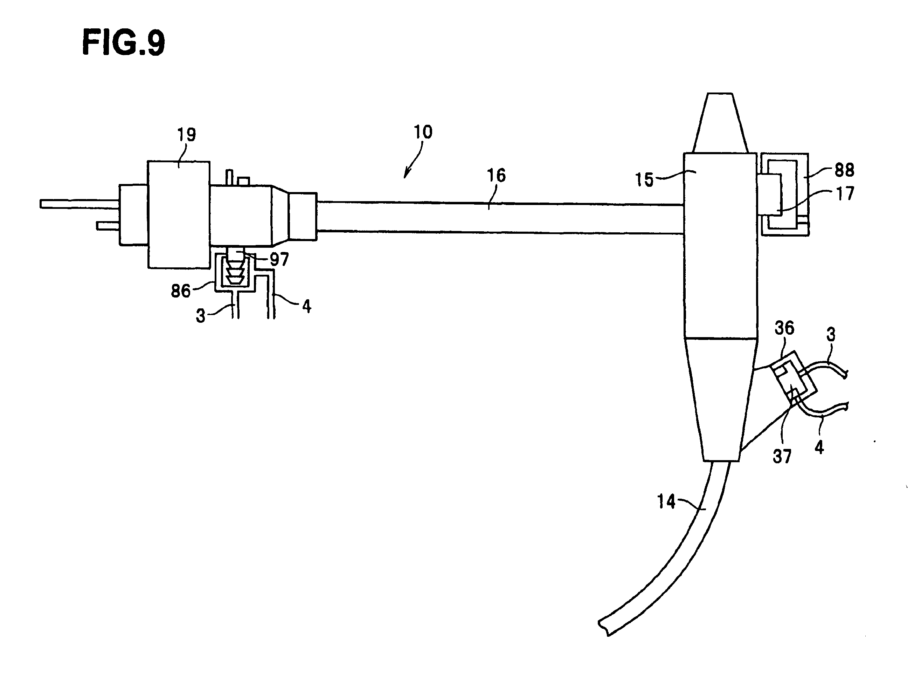

Furthermore, in the structure of an endoscope, a treatment instrument insertion pipe sleeve 37 is provided at a position part way from the operation section 15 to the insertion portion 14, and the conduit is bifurcated. In order to clean/disinfect the inside of the treatment instrument insertion pipe sleeve 37, as illustrated in Fig. 9, an endoscope connection portion 6, 26, 36 or 86 may be connected to both the pipe sleeve 97 and the pipe sleeve 37. Fig. 9 illustrates an example in which the endoscope connection portion 86 is connected to the pipe sleeve 97 and the endoscope connection portion 36 is connected to the pipe sleeve 37.

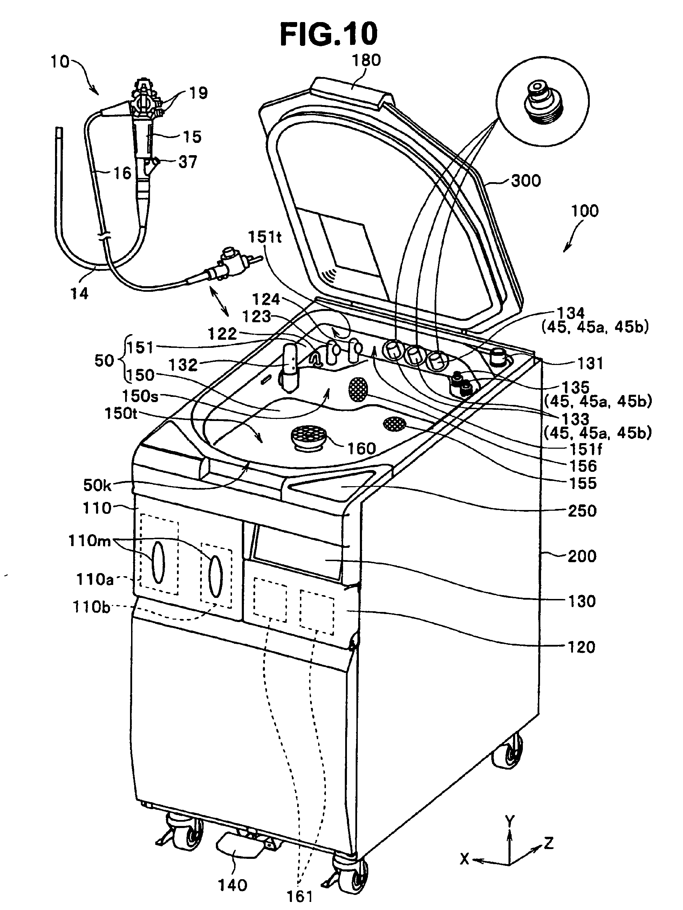

Next, an example of a fluid supply apparatus 100, for example, an endoscope cleaning/disinfecting apparatus to which the fluid supply apparatus connection portion 5, 5a or 5b of the endoscope cleaning/disinfecting apparatus connection tool 1 described above with reference to Figs. 1 to 5 is connectable will be described with reference to Fig. 10. Fig. 10 is a perspective view illustrating an example of an endoscope cleaning/disinfecting apparatus together with an endoscope.

As illustrated in Fig. 10, an endoscope cleaning/disinfecting apparatus 100 is an apparatus for cleaning/disinfecting an endoscope 10 after use, and includes a main portion that includes an apparatus body 200, and a top cover 300 provided above the apparatus body 200, the top cover 300 being a lid body openably/closably connected to the apparatus body 200 via, for example, a non-illustrated hinge.

A non-illustrated container portion is provided at, for example, a left half portion in a width direction X of an upper portion in a height direction Y of the apparatus body 200 at a front face in the Figure of the apparatus body 200 (hereinafter referred to as "front face"), which is near to an operator of the apparatus body 200, and a detergent/alcohol tray 110 which can be pulled out forward of the apparatus body 200 is disposed in the container portion. Hereinafter, a direction in the Figure connecting the front face and a rear face of the apparatus body 200 is referred to a depth direction Z.

The detergent/alcohol tray 110 accommodates a detergent tank 110a storing a cleaning agent used for cleaning the endoscope 10, and an alcohol tank 110b storing an alcohol, which is a liquid used for drying the endoscope 10 after cleaning/disinfection. The detergent/alcohol tray 110 can be pulled out forward in the depth direction Z, enabling liquids to be charged in the respective tanks 10a and 110b in a predetermined manner.

The detergent/alcohol tray 110 is provided with two window portions 110m, whereby the operator can check a remaining amount of the cleaning agent charged in the tank 110a and a remaining amount of the alcohol charged in the tank 110b, respectively. The cleaning agent is a concentrated detergent to be diluted so as to have a predetermined concentration using tap water subjected to filtration processing by a non-illustrated water supply filter.

Also, for example, a cassette tray 120 is disposed at a right half portion in the width direction X of the upper portion in the height direction Y at the front face of the apparatus body 200 so that the cassette tray 120 can be pulled out forward in the depth direction Z of the apparatus body 200. The cassette tray 120 accommodates a disinfectant solution cassette 161 used when the endoscope 10 is disinfected.

The cassette tray 120 can be pulled out, enabling the disinfectant solution cassette 161 to be set in a predetermined manner.

Furthermore, a sub operation panel 130 including, e.g., a display for cleaning/disinfection time and an instruction button for heating a disinfectant solution is disposed at a portion above the cassette tray 120 in the height direction Y at the front face of the apparatus body 200.

Furthermore, a pedal switch 140 for opening the top cover 300, which is closed at the upper portion of the apparatus body 200, upward via the operator's operation to step on the pedal switch 140, is disposed at a lower portion in the height direction Y at front face in the Figure of the apparatus body 200.

Furthermore, main operation panels 250 are provided at positions on, for example, the front face side near to the operator in the depth direction Z, close to opposite ends in the width direction X of an upper face in the height direction Y of the apparatus body 200. The main operation panels 250 include, e.g., a switch for starting an operation to clean/disinfect the apparatus body 200, setting switches such as cleaning/disinfection mode selection switches, and a switch for selecting whether any one or both of the endoscope conduit 18 and the pipe sleeve 17 is cleaned/disinfected.

Also, a water supply hose connection port 131 for supplying tap water to the apparatus body 200 is disposed on the rear side in the depth direction Z facing the front side near to the operator on the upper face in the height direction Y of the apparatus body 200. A water supply hose connected to a faucet is connected to the water supply hose connection port 131. Furthermore, a mesh filter for filtrating tap water may be disposed at the water supply hose connection port 131.

Furthermore, a cleaning/disinfecting bath 50 that can accommodate the endoscope 10 is provided at a substantial center portion of the upper face in the height direction Y of the apparatus body 200. The cleaning/disinfecting bath 50 includes an endoscope accommodating opening that is opened/closed by the top cover 300. The cleaning/disinfecting bath 50 includes a bath body 150 and a terrace portion 151 circumferentially provided so as to be continuous with an outer circumferential edge of the endoscope accommodating opening of the bath body 150.

The bath body 150 can accommodate the endoscope 10 after use when the endoscope 10 after use is cleaned/disinfected, and a drain port 155 for discharging, e.g., the cleaning solution, water, the alcohol and/or the disinfectant solution supplied to the bath body 150 from the bath body 150 or returning the disinfectant solution to a disinfectant solution tank is provided at a bottom face 150t, which is an inner surface of a bath of the bath body 150.

Furthermore, a circulation port 156 is provided at an arbitrary position on a circumferential side face 150s, which is an inner surface of the bath of the bath body 150. The circulation port 156 is provided to supply, e.g., the cleaning solution, the water or the disinfectant solution supplied to the bath body 150 to an endoscope conduit 18 included inside the endoscope 10, via non-illustrated means, or supply, e.g., the cleaning solution, the water and/or the disinfectant solution again to the bath body 150 from a water supply circulation nozzle 124 via, e.g., a mesh filter. A mesh filter for filtrating, e.g., the cleaning solution may be provided at the circulation port 156.

The above-described circulation port 156 may be provided at the bottom face 150t of the bath body 150. Provision of the circulation port 156 at the bottom face 150t of the bath body 150 enables advancement of a timing for supplying, e.g., the cleaning solution, the water or the disinfectant solution to the respective conduits in the endoscope 10 or again to the bath body 150. Furthermore, provision of the circulation port 156 at the bottom face 150t also provides an advantage in that when a user changes a mesh filter provided at the circulation port 156 to another, the operator can easily approach the mesh filter.

A cleaning case 160 is disposed at a substantial center portion of the bottom face 150t of the bath body 150 of the cleaning/disinfecting bath 50.

The cleaning case 160 receives buttons such as respective scope switches 19 for the endoscope 10 and removable parts provided together with the endoscope 10. Consequently, the respective buttons and the removed parts are cleaned/disinfected together with the endoscope 10.

A cover-equipped liquid-level sensor 132 for detecting a level of, e.g., the cleaning solution, the water or the disinfectant solution supplied to the bath body 150 is provided at an arbitrary position at the side face 150s of the bath body 150.

A detergent nozzle 122 and a disinfectant solution nozzle 123 are disposed at a face other than a terrace surface 151t of the terrace portion 151, that is, a face parallel to the bottom face 150t of the bath body 150. The detergent nozzle 122 is provided to supply the cleaning agent, which is diluted so as to have a predetermined concentration using tap water, from the detergent tank 110a to the bath body 150 by means of a non-illustrated detergent pump. The disinfectant solution nozzle 123 is provided to supply the disinfectant solution from the disinfectant solution tank, which will be described later, to the bath body 150 by means of a disinfectant solution supply pump.

Furthermore, a water supply and circulation nozzle 124 is disposed at the face of the terrace portion 151 that is parallel to the bottom face 150t of the bath body 150. The water supply and circulation nozzle 124 is provided to supply the water to the bath body 150, or supply, e.g., the cleaning solution, the water or the disinfectant solution sucked from the circulation port 156 of the bath body 150 again to the bath body 150.

The detergent nozzle 122, the disinfectant solution nozzle 123 and the water supply and circulation nozzle 124 may be disposed at the terrace surface 151t.

Furthermore, a plurality of, here, two ports 133 for air/water-delivery or a forceps opening for supplying, e.g., the cleaning solution, the water, the alcohol, the disinfectant solution or air to the endoscope conduit 18, a forceps elevation port 134, and water leakage detection ports 135 are disposed at a face 151 f on the side facing a position 50k near to the operator of the terrace surface 151t t of the terrace portion 151. These ports 133 to 134 correspond to the ports 45, 45a and 45b described with reference to Figs. 1 to 5, respectively.

Furthermore, the pipe sleeve 17 of the operation section 15 of the endoscope 10 described above with reference to Figs. 1 to 4 is exposed after the respective scope switches 19 are removed from the operation section 15.

The configuration of the endoscope cleaning/disinfecting apparatus is not limited to the configuration in Fig. 10, and may be any configuration as long as such configuration includes ports corresponding to the ports 45, 45a and 45b.

## Claims

1. An endoscope cleaning/disinfecting system in which an endoscope (10) and a fluid supply apparatus (100) are connected through an endoscope cleaning/disinfecting apparatus connection tool (1), the endoscope cleaning/disinfecting apparatus connection tool (1) comprising:
a first introduction port (3i) which is an opening;
a second introduction port (4i) which is an opening independent of the first introduction port (3i);
a first fluid passage portion (3) which is in communication with the first introduction port (3i) and allows a fluid to pass through;
a second fluid passage portion (4) which is in communication with the second introduction port (4i) and allows a fluid to pass through, the second fluid passage portion (4) being a flow passage independent of the first fluid passage portion (3), wherein the first fluid passage portion (3) and the second fluid passage portion (4) each has a tubular shape, and the second fluid passage portion (4) has a diameter larger than a diameter of the first fluid passage portion (3);
a first discharge portion (3t) which is an opening on another end of the first fluid passage portion (3), the first discharge portion (3t) being adapted to discharge the fluid passed through the first fluid passage portion (3); and
a second discharge portion (4t) which is an opening on another end of the second fluid passage portion (4), the second discharge portion (4t) being adapted to discharge the fluid passed through the second fluid passage portion (4),
**characterized in that** the fluid supply apparatus (100) includes a first conduit (43) that supplies a fluid, a first flow control section (63) that is provided in the first conduit (43) and controls a flow rate, a second conduit (44) that supplies a fluid, and a second flow control section (64) provided in the second conduit (44); and
the endoscope cleaning/disinfecting apparatus connection tool (1) further includes:
a fluid supply apparatus connection portion (5) provided with the first introduction port (3i) and the second introduction port (4i) and connected to the fluid supply apparatus (100); and
an endoscope connection portion (6) to be detachably connected to a pipe sleeve (17), the pipe sleeve having an opening portion (17k) that is in communication with a conduit (18) included in the endoscope (10), the endoscope connection portion (6) including the first discharge portion (3t) and the second discharge portion (4t),
wherein the first fluid passage portion (3) is arranged inside the second fluid passage portion (4) and located along the second fluid passage portion (4) and the fluid supply apparatus (100) is adapted to supply the same fluid to the first fluid passage portion (3) and the second fluid passage portion (4),
wherein the first discharge portion (3t) is configured to be insertable into the opening portion (17k), and when the endoscope connection portion (6) is connected to the pipe sleeve (17) and the first discharge portion (3t) is inserted into the opening portion (17k), the first discharge portion (3t) is adapted to discharge the fluid into the conduit (18), and
when the endoscope connection portion (6) is connected to the pipe sleeve (17), the second discharge portion (4t) is arranged at a circumference of the opening portion (17k) and is adapted to discharge the fluid to the circumference of the opening portion (17k).

2. The endoscope cleaning/disinfecting system according to claim 1,
wherein the first flow control section (63) and the second flow control section (64) each include an openable/closable valve element, and
wherein the endoscope cleaning/disinfecting system further comprises a control section (40) that performs control to open/close the first flow control section (63) and the second flow control section (64) to perform control to supply the fluid to at least one of the first conduit (43) and the second conduit (44), and controls an amount of supply of the fluid flowing in the first conduit (43) and an amount of supply of the fluid flowing in the second conduit (44).

## Patentansprüche

1. Endoskop-Reinigungs-/Desinfektionssystem, in dem ein Endoskop (10) und eine Fluidzufuhreinrichtung (100) durch ein Endoskop-Reinigungs-/Desinfektionsvorrichtungsverbindungswerkzeug (1) miteinander verbunden sind, wobei das Endoskop-Reinigungs-/Desinfektionsvorrichtungsverbindungswerkzeug (1) umfasst:
einen ersten Einlassanschluss (3i), der eine Öffnung ist;
einen zweiten Einlassanschluss (4i), der eine von dem ersten Einlassanschluss (3i) unabhängige Öffnung ist;
einen ersten Fluidleitungsabschnitt (3), der mit dem ersten Einlassanschluss (3i) in Kommunikation steht und den Durchfluss eines Fluids ermöglicht;
einen zweiten Fluidleitungsabschnitt (4), der mit dem zweiten Einlassanschluss (4i) in Kommunikation steht und den Durchfluss eines Fluids ermöglicht, wobei der zweite Fluidleitungsabschnitt (4) eine von dem ersten Fluidleitungsabschnitt (3) unabhängige Strömungsleitung ist, wobei der erste Fluidleitungsabschnitt (3) und der zweite Fluidleitungsabschnitt (4) jeweils rohrförmig ausgebildet sind, und der zweite Fluidleitungsabschnitt (4) einen Durchmesser hat, der größer ist als der Durchmesser des ersten Fluidleitungsabschnitts (3);
einen ersten Auslassabschnitt (3t), der eine Öffnung an einem anderen Ende des ersten Fluidleitungsabschnitt (3) ist, wobei der erste Auslassabschnitt (3t) dazu eingerichtet ist, das durch den ersten Fluidleitungsabschnitt (3) geleitete Fluid abzulassen; und
einen zweiten Auslassabschnitt (4t), der eine Öffnung an einem anderen Ende des zweiten Fluidleitungsabschnitts (4) ist, wobei der zweite Auslassabschnitt (4t) dazu eingerichtet ist, das durch den zweiten Fluidleitungsabschnitt (4) geleitete Fluid abzulassen,
**dadurch gekennzeichnet, dass** die Fluidzufuhreinrichtung (100) eine erste Leitung (43), die ein Fluid zuführt, einen ersten Strömungssteuerungsabschnitt (63), der in der ersten Leitung (43) vorgesehen ist und eine Strömungsrate steuert, eine zweite Leitung (44), die ein Fluid zuführt, und einen zweiten Strömungssteuerungsabschnitt (64) umfasst, der in der zweiten Leitung (44) vorgesehen ist; und
dass das Endoskop-Reinigungs-/Desinfektionsvorrichtungsverbindungs-werkzeug (1) ferner umfasst:
einen Fluidzufuhreinrichtungsverbindungsabschnitt (5), der mit dem ersten Einlassanschluss (3i) und dem zweiten Einlassanschluss (4i) versehen und mit der Fluidzufuhreinrichtung (100) verbunden ist; und
einen Endoskopverbindungsabschnitt (6), der lösbar mit einer Rohrhülse (17) verbunden ist, wobei die Rohrhülse einen Öffnungsabschnitt (17k) hat, der mit der einer Leitung (18) in dem Endoskop (10) in Kommunikation steht, und wobei der Endoskopverbindungsabschnitt (6) den ersten Auslassabschnitt (3t) und den zweiten Auslassabschnitt (4t) umfasst,
wobei der erste Fluidleitungsabschnitt (3) innerhalb des zweiten Fluidleitungsabschnitts (4) positioniert und entlang des zweiten Fluidleitungsabschnitts (4) angeordnet ist und die Fluidzufuhreinrichtung (100) dazu eingerichtet ist, dem ersten Fluidleitungsabschnitt (3) und dem zweiten Fluidleitungsabschnitt (4) das gleiche Fluid zuzuführen,
wobei der erste Auslassabschnitt (3t) so ausgeführt ist, dass er in den Öffnungsabschnitt (17k) einführbar ist, und wenn der Endoskopverbindungsabschnitt (6) mit der Rohrhülse (17) verbunden ist und der erste Auslassabschnitt (3t) in den Öffnungsabschnitt (17k) eingeführt ist, der erste Auslassabschnitt (3t) dazu eingerichtet ist, das Fluid in die Leitung (18) abzulassen, und
wenn der Endoskopverbindungsabschnitt (6) mit der Rohrhülse (17) verbunden ist, der zweite Auslassabschnitt (4t) entlang eines Umfangs des Öffnungsabschnitts (17k) angeordnet und dazu eingerichtet ist, das Fluid zu dem Umfang des Öffnungsabschnitts (17k) abzulassen.

2. Endoskop-Reinigungs-/Desinfektionssystem nach Anspruch 1,
wobei der erste Strömungssteuerungsabschnitt (63) und der zweite Strömungssteuerungsabschnitt (64) jeweils ein öffnungsfähiges/schließbares Ventilelement umfassen, und
wobei das Endoskop-Reinigungs-/Desinfektionssystem ferner einen Steuerungsabschnitt (40) umfasst, der eine Steuerung zum Öffnen/Schließen des ersten Strömungssteuerungsabschnitts (63) und des zweiten Strömungssteuerungsabschnitts (40) durchführt, um eine Steuerung der Zufuhr des Fluids zu der ersten Leitung (43) und/oder der zweiten Leitung (44) durchzuführen, und der einen Betrag der Zufuhr des in der ersten Leitung (43) fließenden Fluids und einen Betrag der Zufuhr des in der zweiten Leitung (44) fließenden Fluids steuert.

## Revendications

1. Système de nettoyage/désinfection d'endoscope dans lequel un endoscope (10) et un appareil (100) d'alimentation en fluide sont connectés par l'intermédiaire d'un outil (1) de connexion d'appareil de nettoyage/désinfection d'endoscope, l'outil (1) de connexion d'appareil de nettoyage/désinfection d'endoscope comprenant :
un premier orifice d'introduction (3i) qui est une ouverture ;
un deuxième orifice d'introduction (4i) qui est une ouverture indépendante du premier orifice d'introduction (3i) ;
une première partie (3) de passage de fluide qui est en communication avec le premier orifice d'introduction (3i) et permet à un fluide de passer à travers ;
une deuxième partie (4) de passage de fluide qui est en communication avec le deuxième orifice d'introduction (4i) et permet à un fluide de passer à travers, la deuxième partie (4) de passage de fluide étant un passage (3) d'écoulement indépendant de la première partie de passage de fluide, dans lequel la première partie (3) de passage de fluide et la deuxième partie (4) de passage de fluide présentent chacune une forme tubulaire, et la deuxième partie (4) de passage de fluide présente un diamètre plus grand qu'un diamètre de la première partie (3) de passage de fluide ;
une première partie d'évacuation (3t) qui est une ouverture sur une autre extrémité de la première partie (3) de passage de fluide, la première partie d'évacuation (3t) étant adaptée pour évacuer le fluide ayant traversé la première partie (3) de passage de fluide ; et
une deuxième partie d'évacuation (4t) qui est une ouverture sur une autre extrémité de la deuxième partie (4) de passage de fluide, la deuxième partie d'évacuation (4t) étant adaptée pour évacuer le fluide ayant traversé la deuxième partie (4) de passage de fluide,
**caractérisé en ce que** l'appareil (100) d'alimentation en fluide comprend un premier conduit (43) qui délivre un fluide, une première section (63) de commande d'écoulement qui est prévue dans le premier conduit (43) et commande un débit d'écoulement, un deuxième conduit (44) qui délivre un fluide, et une deuxième section (64) de commande d'écoulement prévue dans le deuxième conduit (44) ; et
l'outil (1) de connexion d'appareil de nettoyage/désinfection d'endoscope comprend en outre :
une partie (5) de connexion d'appareil d'alimentation en fluide munie du premier orifice d'introduction (3i) et du deuxième orifice d'introduction (4i) et connectée à l'appareil (100) d'alimentation en fluide ; et
une partie (6) de connexion d'endoscope destinée à être connectée de manière détachable à un manchon de tuyau (17), le manchon de tuyau comportant une partie d'ouverture (17k) qui est en communication avec un conduit (18) contenu dans l'endoscope (10), la partie (6) de connexion d'endoscope incluant la première partie d'évacuation (3t) et la deuxième partie d'évacuation (4t),
dans lequel la première partie (3) de passage de fluide est disposée à l'intérieur de la deuxième partie (4) de passage de fluide et placée le long de la deuxième partie (4) de passage de fluide et l'appareil (100) d'alimentation en fluide est adapté pour délivrer le même fluide vers la première partie (3) de passage de fluide et la deuxième partie (4) de passage de fluide,
dans lequel la première partie d'évacuation (3t) est configurée pour pouvoir être insérée dans la partie d'ouverture (17k), et lorsque la partie (6) de connexion d'endoscope est connectée au manchon de tuyau (17) et que la première partie d'évacuation (3t) est insérée dans la partie d'ouverture (17k), la première partie d'évacuation (3t) est adaptée pour évacuer le fluide dans le conduit (18), et
lorsque la partie (6) de connexion d'endoscope est connectée au manchon de tuyau (17), la deuxième partie d'évacuation (4t) est disposée au niveau d'une circonférence de la partie d'ouverture (17k) et est adaptée pour évacuer le fluide vers la circonférence de la partie d'ouverture (17k).

2. Système de nettoyage/désinfection d'endoscope selon la revendication 1,
dans lequel la première section (63) de commande d'écoulement et la deuxième section (64) de commande d'écoulement comprennent chacune un élément de valve pouvant s'ouvrir/se fermer, et
dans lequel le système de nettoyage/désinfection d'endoscope comprend en outre une section de commande (40) qui exécute une commande pour ouvrir/fermer la première section (63) de commande d'écoulement et la deuxième section (64) de commande d'écoulement pour exécuter une commande pour délivrer le fluide vers au moins l'un parmi le premier conduit (43) et le deuxième conduit (44), et commande une quantité d'alimentation en fluide circulant dans le premier conduit (43) et une quantité d'alimentation en fluide circulant dans le deuxième conduit (44).
